(19) Europäisches Patentamt
European Patent Office
Office européen des brevets

(11) Veröffentlichungsnummer: **0 196 592**
**B1**

(12) **EUROPÄISCHE PATENTSCHRIFT**

(45) Veröffentlichungstag der Patentschrift: **07.12.88**

(51) Int. Cl.⁴: **C 07 D 263/58**

(21) Anmeldenummer: **86104032.7**

(22) Anmeldetag: **24.03.86**

(54) **Verfahren zur Herstellung von 2-Merkaptobenzoxazolen.**

(30) Priorität: **04.04.85 DE 3512295**

(43) Veröffentlichungstag der Anmeldung:
**08.10.86 Patentblatt 86/41**

(45) Bekanntmachung des Hinweises auf die Patenterteilung:
**07.12.88 Patentblatt 88/49**

(84) Benannte Vertragsstaaten:
**BE CH DE FR GB IT LI NL**

(56) Entgegenhaltungen:
**CHEMICAL ABSTRACTS, Band 49, Nr. 17, 10. September 1955, Columbus, Ohio, USA LEON KATZ AND MURRAY S. COHEN "Benzoxazole derivatives. I. 2-Mercaptobenzoxazoles" Spalte 11623, Zusammenfassun-g**
**CHEMICAL ABSTRACTS, Band 45, Nr. 1, 10. Jänner 1951, Columbus, Ohio, USA J.A. VAN ALLAN AND B. D. DEACON "2-Mercaptobenzimidazole" Spalte 158, Zusammenfassung-c**
**CHEMICAL ABSTRACTS, Band 99, Nr. 23, 5. Dezember 1983, Columbus, Ohio, USA YAMATO, MASOTOSHI; TEKEUCHI, YASUO; HASHIGAKI, KINIKO; HATTORI, KYOKO; MUROGA, EIKO; HIROTA, TAKASHI**

(73) Patentinhaber: **CASSELLA Aktiengesellschaft, Hanauer Landstrasse 526, D-6000 Frankfurt am Main 61 (DE)**

(72) Erfinder: **Kussmaul, Ulrich, Dr., Tannenweg 39a, D-6367 Karben 1 (DE)**
Erfinder: **Langer, Manfred, Dr., Birsteiner Strasse 47, D-6000 Frankfurt am Main 61 (DE)**
Erfinder: **Reh, Kuno, Dr., Lauterbacher Strasse 14, D-6000 Frankfurt am Main 61 (DE)**
Erfinder: **Becherer, Johannes, Dr., Weidenseestrasse 8, D-6457 Maintal-Bischofsheim (DE)**
Erfinder: **Wille, Herbert, Dr., Hünfelder Strasse 16, D-6000 Frankfurt am Main 61 (DE)**
Erfinder: **Müller, Rolf, Dr., Dornbachweg 3, D-6367 Karben 1 (DE)**

(74) Vertreter: **Urbach, Hans-Georg, Dr., Hanauer Landstrasse 526, D-6000 Frankfurt am Main 61 (DE)**

(56) Entgegenhaltungen: (Fortsetzung)
**"Synthesis of 3-substituted benzoxazoline-2-thiones" Seite 744, Spalte 2, Zusammenfassung-Nr. 194850q**
**CHEMICAL ABSTRACTS, Band 78, Nr. 15, 16. April 1973, Columbus, Ohio, USA WAGNER, G.; LEISTNER, S. "Cleavage of 3-benzoylbenzoxazolinones and their monothioxo isologs with hydroxide and methoxide" Seite 470, Spalte 2, Zusammenfassung-Nr. 97 534y**
**CHEMICAL ABSTRACTS, Band 93, Nr. 25, 22. Dezember 1980, Columbus, Ohio, USA FOYE, WILLIAM O.; ABOOD, NORMAN; KAUFMAN, JOEL M.; KIM, YOUNG-HO; PATEL, BHUPENDRA R. "A direct synthesis of heterocyclic thiols" Seite 851, Spalte 1, Zusammenfassung-Nr. 239 367g**

**EP 0 196 592 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von 2-Merkaptobenzoxazolen der Formel I

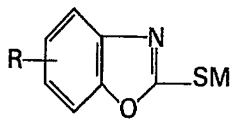

(I)

worin R Wasserstoff oder Halogen und M Wasserstoff oder ein Alkalimetallatom bedeutet. Die Verbindungen der Formel I sind wertvolle Vorprodukte zur Herstellung hochwirksamer Herbizide. Es besteht daher ein dringendes Bedürfnis nach einem kostengünstigen und gleichzeitig umweltfreundlichen Verfahren zur Herstellung dieser Verbindungen.

2-Merkaptobenzoxazol ist bereits 1876 von Dünner (Ber. 9, [1876] S. 465) beschrieben worden. Die Herstellung von 2-Merkaptobenzoxazol und seiner Derivate erfolgt nach Verfahren, die prinzipiell auf der Reaktion zwischen o-Aminophenol und Alkalialkylxanthaten beruhen. Dabei kann das Alkalialkylxanthat entweder als solches eingesetzt, oder es kann im Reaktionsgemisch selbst aus Alkali, einem niederen Alkanol und Schwefelkohlenstoff erzeugt werden.

Den bekannten Verfahren haften jedoch erhebliche Nachteile an, die ihre Überführung in den grosstechnischen Massstab erheblich erschweren. So ist das Arbeiten mit Schwefelkohlenstoff nur in speziellen explosionsgeschützten Apparaturen möglich, wobei stets ein schwer kalkulierbares Restrisiko bleibt. Auch die Verwendung grösserer Mengen von Alkanolen ist ungünstig, da sie möglichst quantitativ recyclisiert werden müssen und Verluste zu einer Belastung des Abwassers, verbunden mit erhöhten Abwasserreinigungskosten führen.

Aus der europäischen Patentanmeldung 66248 ist ein Verfahren bekannt nach dem sich Verbindungen der Formel I in guten Ausbeuten herstellen lassen. Aber auch diesem Verfahren haften noch Nachteile an, die z. T. erst bei der grosstechnischen Durchführung sichtbar werden und diese erheblich erschweren. So wird auch hier Alkalialkylxanthat mit Aminophenolen umgesetzt, wobei die Reaktion in einem organischen Lösungsmittel oder auch in Wasser erfolgen kann. Die hierbei erforderliche Dosierung der grosstechnisch verfügbaren festen Alkalialkylxanthate erfordert einen besonderen technischen Aufwand und besondere Umweltschutzmassnahmen wegen der Bildung von Stäuben.

Die Durchführung des Verfahrens nach dem Stand der Technik in einem organischen Lösungsmittel erfordert einen erhöhten Aufwand zur Regenerierung dieser Lösungsmittel und zur Entfernung von Alkoholen. Bei der Durchführung in Wasser fällt ein mit Alkoholen (aus den Xanthaten) belastetes Abwasser an, das durch physikalische, chemische oder biologische Methoden gereinigt werden muss. Z. B. ergibt dieser Alkohol eine erhöhte CSB/BSB5-Belastung und erfordert einen zusätzlichen Aufwand bei der biologischen Abwasserbehandlung. Ferner hat die grosstechnische Durchführung des Verfahrens gemäss dem Stand der Technik gezeigt, dass die Abluft neben Schwefelwasserstoff stark geruchsbelästigende Niederalkylmerkaptane enthält, die entweder bereits in den eingesetzten Xanthaten als Merkaptide enthalten sind oder sich aus diesen bilden.

Es wurde nun gefunden, dass sich die Nachteile der bisher bekannten Herstellungsverfahren vermeiden lassen, wenn man zur Herstellung der Verbindungen der Formel I ein Aminophenol der Formel II

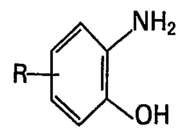

(II)

worin R die oben genannten Bedeutungen hat, bzw. dessen Metallsalz, insbesondere dessen Alkalimetallsalz mit einem Alkalitrithiocarbonat in wässriger Lösung umsetzt.

Die Reihenfolge, in der die Reaktionskomponenten vereinigt werden, ist für das Verfahren unerheblich. So kann eine Lösung oder Suspension des Aminophenols der Formel II bzw. dessen Metallsalz in Wasser vorgelegt und während der Reaktion das Alkalitrithiocarbonat, vorzugsweise in Form einer wässrigen Lösung, zudosiert werden. Es ist aber auch der umgekehrte Vorgang durchführbar, wenn eine betriebliche Notwendigkeit dafür besteht. Man kann jedoch auch alle Reaktionskomponenten, nämlich Aminophenol bzw. dessen Salz, Alkalitrithiocarbonat und Wasser, zuerst mischen und dann bei der gewünschten Reaktionstemperatur halten. Die Reaktionstemperatur ist in weiten Grenzen variabel und richtet sich nach den im Betrieb gegebenen Möglichkeiten. So bringt die Durchführung der Reaktion bei Normaltemperatur von 20°C zwar eine Einsparung von Heizkosten mit sich und ist in allereinfachsten Apparaturen durchzuführen, führt jedoch zwangsläufig zu einer drastischen Verlängerung der Reaktionsdauer. Andererseits kann bei Arbeiten bei 150°C eine sehr kurze Reaktionszeit erzielt werden, wenn eine entsprechende Apparatur vorhanden und eine gewisse Qualitätsminderung des Produkts akzeptabel ist. Vorzugsweise arbeitet man im Temperaturbereich von 50 bis 120°C.

Es versteht sich, dass man bei der Durchführung der Reaktion oberhalb zirka 100°C in einem geschlossenen Gefäss unter dem entsprechenden Dampfdruck des Reaktionsgemisches arbeitet.

In der Regel wird pro Mol Aminophenol der Formel II 1,00 bis 1,2, vorzugsweise 1,00 bis 1,1, bei grösseren Ansätzen insbesondere 1,02 bis 1,06 Mol Alkalitrithiocarbonat eingesetzt.

Besonders bequem und ökonomisch lässt sich das erfindungsgemässe Verfahren bei Einsatz der technischen Alkalitrithiocarbonate, insbesondere in Form von handelsüblichen wässrigen Lösungen, ausführen.

Während der Reaktion stellt sich von selbst ein

alkalischer pH-Wert ein, der jedoch nicht hoch genug ist, um das Entweichen von gebildetem $H_2S$ zu verhindern. Wie die Reaktionsgleichung der dem erfindungsgemässen Verfahren zugrunde liegenden Umsetzung zeigt, werden bei der Bildung des Oxazolkerns zwei Mol sulfidischer Schwefel frei, von denen ein Teil im Gleichgewicht als $H_2S$ vorliegt und aus dem Reaktionsgemisch freigesetzt wird. Will man das Auftreten von freiem $H_2S$ verhindern, so kann dem Reaktionsgemisch pro Mol des Aminophenols der Formel II mindestens 1 Grammäquivalente Alkali zugesetzt werden, das den abgespaltenen Schwefelwasserstoff als Sulfid bindet. Zweckmässigerweise wird in diesem Fall mit einem gewissen Alkaliüberschuss gearbeitet. Beim Arbeiten in einem abgeschlossenen System unter geringem Überdruck, beispielsweise bei 0,02 bis 0,2 bar, wie er sich z. B. beim Abschluss des Reaktionskessels durch einen Flüssigkeitsverschluss ergibt, werden üblicherweise 102 bis 110% der theoretisch erforderlichen Alkalimenge eingesetzt. Will man in der Praxis auch bei offener Fahrweise das Auftreten von freiem $H_2S$ während der Reaktion sicher vermeiden, so ist es zweckmässig, 2 bis 3 Grammäquivalente Alkali zuzusetzen. Als Alkali eignen sich vor allem Alkalihydroxide, wie NaOH oder KOH, und Salze der Alkalimetalle mit Säuren, die schwächer sind als $H_2S$, und die daher in der Lage sind, $H_2S$ als Sulfid zu binden.

Nach Ablauf der Reaktion werden in der Regel wässrige Lösungen, bei sehr hoch konzentrierten Ansätzen wässrige Dispersionen der Alkalisalze der Benzoxazolderivate der Formel I erhalten, die in vielen Fällen direkt für weitere Umsetzungen eingesetzt werden können.

Sollen die wässrigen Lösungen nicht direkt weiterverarbeitet werden, so können die erhaltenen 2-Merkaptobenzoxazole der Formel I auch in an sich bekannter Weise isoliert werden. Zu diesem Zweck ist es üblich, die erhaltene wässrige Lösung bzw. Dispersion durch Zusatz einer vorzugsweise anorganischen Säure soweit anzusäuern, dass nach Massgabe des $pK_s$-Wertes des hergestellten Merkaptobenzoxazols eine vollständige Ausfällung des Produkts eintritt (pH zirka 0–4) und, sofern der bei der Reaktion entstandene $H_2S$ durch Alkalizusatz abgefangen worden war, den gegebenenfalls ausgetriebenen Schwefelwasserstoff zu absorbieren. Das beim Ansäuren aus dem Alkalisalz freigesetzte Merkaptobenzoxazolderivat der Formel I fällt aus und kann durch eine fest/flüssig-Trennungsoperation, vorzugsweise durch Filtration, isoliert werden. Das hierbei erhaltene Filtrat ist, im Gegensatz zu dem nach dem Xanthatverfahren erhaltenen, ein nur schwach belastetes Abwasser.

Das erfindungsgemässe Verfahren erlaubt die Durchführung der Reaktion in bequemer Weise in offenen oder geschlossenen Gefässen, drucklos oder unter Druck, diskontinuierlich oder kontinuierlich. Wenn das Reaktionsgemisch genügend alkalisch ist, kann auf eine Absorptionsanlage für Schwefelwasserstoff während der Reaktion somit ganz verzichtet werden.

Besonders wertvoll ist das erfindungsgemässe Verfahren für die Herstellung solcher Verbindungen der Formel I, in denen R Fluor, Chlor oder Brom, insbesondere aber Chlor, oder Wasserstoff bedeutet. Besonders bevorzugt ist die Herstellung solcher Verbindungen der Formel I, in denen R in der 6-Stellung des Benzoxazolsystems steht.

2-Merkaptobenzoxazol wird nach dem erfindungsgemässen Verfahren erhalten, wenn als Ausgangsmaterial der Formel II 2-Aminophenol eingesetzt wird; zur Herstellung von 6-Halogen-2-merkaptobenzoxazol geht man entsprechend von 5-Halogen-2-aminophenolen aus.

Bevorzugte Bedeutungen von M sind Na und K, sowie Wasserstoff.

Besonders bevorzugt werden solche Verbindungen der Formel I hergestellt, in denen mehrere bevorzugte Merkmale vereinigt sind.

Es überrascht den Fachmann, dass das vorteilhafte und umweltfreundliche erfindungsgemässe Verfahren dieselben guten Ausbeuten bei kurzen Reaktionszeiten liefert wie das in der Grosstechnik schwierig durchzuführende Xanthatverfahren, obwohl es theoretisch nach dem Massenwirkungsgesetz erheblich benachteiligt ist.

So wird während der Cyclisierungsreaktion nach dem Xanthatverfahren gemäss EP-OS 66248 eine lebhafte Schwefelwasserstoffentwicklung beobachtet. Damit wird ein Reaktionsprodukt dem Reaktionsgleichgewicht kontinuierlich entzogen, was nach dem Massenwirkungsgesetz den Fortschritt der Reaktion begünstigt.

Überraschenderweise werden beim erfindungsgemässen Verfahren die gewünschten Produkte der Formel I in gleichen Ausbeuten und Reinheiten bei gleicher Reaktionszeit erhalten, obwohl den Reaktionsgemischen im Vergleich zum Xanthatverfahren zusätzlich ein weiteres Mol sulfidischer Schwefel als Trithiocarbonat zugeführt wird, was als ungünstig im Sinne des Massenwirkungsgesetzes gewertet werden muss. Als äusserst überraschend muss gelten, dass die Reaktion sogar dann noch so schnell wie beim Xanthatverfahren abläuft, wenn man durch den Zusatz von Natronlauge die Entwicklung von Schwefelwasserstoff und dessen Entfernung aus dem Reaktionsgemisch während der Reaktion ganz unterdrückt.

Die folgenden Ausführungsbeispiele veranschaulichen das erfindungsgemässe Verfahren.

Beispiel 1:

In einem Rührwerkskolben mit Thermometer und Rücklaufkühler werden 170,8 g 84,1%iges 5-Chlor-2-aminophenol, 336,9 g 48%iges Natriumtrithiocarbonat als wässrige Lösung, 121,6 ml Natronlauge 33°Bé und 250 ml Wasser gemischt, das Reaktionsgefäss mit einer 1 m hohen Salzwassersäule abgesperrt und 3 Stunden unter ständigem Rühren am Rückfluss gekocht. Der Ansatz wird abgekühlt, und durch Zusatz von Schwefelsäure wird in bekannter Weise das erhaltene 6-Chlor-2-merkaptobenzoxazol ausgefällt, abfiltriert und der Filterkuchen mit Wasser salzfrei gewaschen und bei 80°C im Vakuum getrocknet.

Man erhält 179 g 6-Chlor-2-merkaptobenzoxa-

zol, (entsprechend 96% der Theorie) mit einem Schmelzpunkt von 223–224°C.

Wird die Reaktion bei 80°C durchgeführt, so erhält man nach einer Reaktionszeit von 24 Stunden eine Ausbeute von 95% der Theorie; bei 150°C in geschlossener Reaktion verläuft die Cyclisierung in weniger als 1 Stunde, man erhält dabei eine Ausbeute von 90 % der Theorie.

Beispiel 2:
In einem Rührwerkskolben mit Thermometer und Rücklaufkühler werden 170,8 g 84,1 %iges 5-Chlor-2-aminophenol und 350 ml Wasser aufgeschlämmt. Die Suspension wird auf 95 bis 100°C erwärmt und bei dieser Temperatur unter Rühren im Verlauf von 3 Stunden eine Lösung von 336,9 g einer 48gew.%igen wässrigen Natriumtrithiocarbonatlösung zudosiert. Danach wird noch 3 Stunden unter Rückfluss gekocht. Nach Aufarbeitung der erhaltenen Lösung wie im Beispiel 1 erhält man 181 g (97% der Theorie) 6-Chlor-2-merkaptobenzoxazol vom Schmelzpunkt 221 bis 223°C.

Ersetzt man in einem der Beispiele 1 oder 2 das 5-Chlor-2-aminophenol durch 109,1 g reines oder 116,1 g technisches 94%iges 2-Aminophenol, so erhält man gemäss Beispiel 1 98% der Theorie, gemäss Beispiel 2 96% der Theorie 2-Merkaptobenzoxazol vom Schmelzpunkt 192 bis 193°C.

Wird die Reaktion bei 50°C durchgeführt, so muss mehr als 24 Stunden gerührt werden, um eine Ausbeute von mindestens 90% der Theorie zu erhalten.

## Patentansprüche

1. Verfahren zur Herstellung von 2-Merkaptobenzoxazolen der Formel I

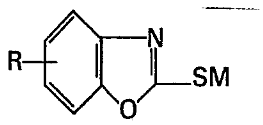

(I)

worin R Wasserstoff oder Halogen und M Wasserstoff oder ein Alkalimetallatom bedeuten, dadurch gekennzeichnet, dass man ein Aminophenol der Formel II

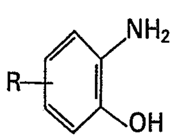

(II)

worin R die oben angegebenen Bedeutungen hat, bzw. dessen Metallsalze, mit einem Alkalitrithiocarbonat in wässriger Lösung umsetzt.

2. Verfahren gemäss Anspruch 1, dadurch gekennzeichnet, dass die Umsetzung bei Temperaturen von 20 bis 150°C durchgeführt wird.

3. Verfahren gemäss den Ansprüchen 1 und 2, dadurch gekennzeichnet, dass die Umsetzung bei Temperaturen von 50 bis 120°C durchgeführt wird.

4. Verfahren gemäss den Ansprüchen 1 bis 3, dadurch gekennzeichnet, dass die Umsetzung in Gegenwart von zusätzlichen Basen durchgeführt wird.

5. Verfahren gemäss den Ansprüchen 1 bis 4, dadurch gekennzeichnet, dass man als Aminophenol der Formel II 2-Aminophenol oder 5-Chlor-2-aminophenol einsetzt.

## Claims

1. Process for the preparation of 2-mercaptobenzoxazoles of the formula I

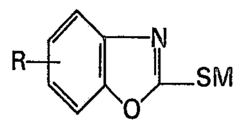

(I)

wherein R denotes hydrogen or halogen and M denotes hydrogen or an alkali metal atom, characterised in that an aminophenol of the formula II

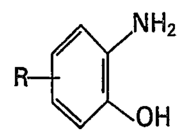

(II)

wherein R has the abovementioned meanings, or a metal salt thereof, is reacted with an alkali metal trithiocarbonate in aqueous solution.

2. Process according to Claim 1, characterised in that the reaction is carried out at temperatures from 20 to 150°C.

3. Process according to Claims 1 and 2, characterised in that the reaction is carried out at temperatures from 50 to 120°C.

4. Process according to Claims 1 to 3, characterised in that the reaction is carried out in the presence of additional bases.

5. Process according to Claims 1 to 4, characterised in that 2-aminophenol or 5-chloro-2-aminophenol is employed as the aminophenol of the formula II.

## Revendications

1. Procédé pour préparer des mercapto-2 benzoxazoles répondant à la formule I:

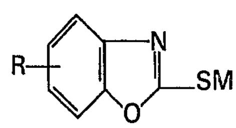

(I)

dans laquelle: R représente l'hydrogène ou un halogène et M représente l'hydrogène ou un atome de métal alcalin, procédé caractérisé en ce qu'on fait réagir un aminophénol répondant à la formule II:

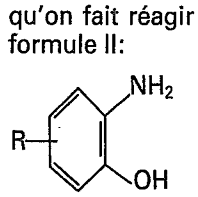

(II)

dans laquelle R a la signification donnée ci-dessus, ou un sel de métal de celui-ci, avec un trithiocarbonate de métal alcalin, en solution aqueuse.

2. Procédé selon la revendication 1 caractérisé en ce qu'on effectue la réaction à des températures de 20 à 150°C.

3. Procédé selon l'une des revendications 1 et 2, caractérisé en ce qu'on effectue la réaction à des températures de 50 à 120°C.

4. Procédé selon l'une quelconque des revendications 1 à 3, caractérisé en ce qu'on effectue la réaction en présence de bases supplémentaires.

5. Procédé selon l'une quelconque des revendications 1 à 4, caractérisé en ce qu'on utilise, comme aminophénol de formule II, l'amino-2 phénol ou le chloro-5 amino-2 phénol.